Europäisches Patentamt

European Patent Office

Office européen des brevets

(19)

(11) Numéro de publication: **0 221 796**

**A1**

(12)

# DEMANDE DE BREVET EUROPEEN

(21) Numéro de dépôt: **86402116.7**

(22) Date de dépôt: **26.09.86**

(51) Int. Cl.⁴: **G 01 N 29/02**
**G 01 N 33/20**

(30) Priorité: **30.09.85 FR 8514466**

(43) Date de publication de la demande:
**13.05.87 Bulletin 87/20**

(84) Etats contractants désignés:
**DE GB IT**

(71) Demandeur: **NOVATOME**
**Tour Fiat 1, place de la Coupole**
**F-92400 - Courbevoie(FR)**

(72) Inventeur: **Lebaud, Patrice**
**60 Rue Marie Fichet**
**F-92140 Clamart(FR)**

(72) Inventeur: **Isnardon, Gérald**
**17 Rue Saint Guillaume**
**F-92400 Courbevoie(FR)**

(74) Mandataire: **Polus, Camille et al,**
**c/o Cabinet Lavoix 2, Place d'Estienne d'Orves**
**F-75441 Paris Cedex 09(FR)**

(54) **Procédé et dispositif de détection par ultrasons de bulles de gaz dans un métal liquide.**

(57) On envoie un faisceau d'ultrasons dans le métal liquide depuis un point d'émission (3) situé à l'extérieur de l'enveloppe (1), on capte le faisceau d'ultrasons à l'extérieur de l'enveloppe (1) après qui'il ait traversé le métal liquide, on compare l'amplitude des ondes ultrasonores captées à l'amplitude des ondes ultrasonores émises et on détermine l'atténuation des ultrasons produite par la traversée du métal liquide renfermant éventuellement des bulles de gaz (2). Le dispositif suivant l'invention comporte un guide d'ondes (4) lié métallurgiquement à l'enveloppe (1). Une unité de traitement (10) reliée aux transducteurs d'ultrasons (3, 6) permet de mesurer l'atténuation.

FIG. 1

EP 0 221 796 A1

Croydon Printing Company Ltd.

L'invention concerne un procédé et un dispositif de détection par ultrasons de bulles de gaz dans un métal liquide contenu dans une enveloppe.

Les métaux liquides, en particulier les métaux liquides en circulation, peuvent contenir des bulles de gaz de taille plus ou moins importante qui sont entraînées par le liquide dans toutes les parties de l'installation industrielle où ces métaux sont utilisés. L'origine et la nature de ces gaz peuvent être très diverses mais généralement ces gaz perturbent, parfois de façon extrèmement grave, le fonctionnement de l'installation.

Dans le cas des réacteurs nucléaires a neutrons rapides refroidis par du sodium liquide, le gaz contenu dans le sodium peut être de l'hydrogene provenant du générateur de vapeur, dans le cas où ce générateur présente une petite fuite, l'eau de refroidissement étant décomposée par le sodium.

Ce gaz peut être également de l'argon ou autre gaz inerte qui sert d'atmosphère de couverture au sodium et qui peut être entraîné par une pompe de circulation.

Ce gaz peut être constitué egalement par des bulles de vapeur du métal liquide produites par la cavitation d'une pompe.

Dans le premier cas, une détection rapide des bulles d'hydrogene est necessaire pour détecter une fuite éventuelle dans le générateur de vapeur qui peut nécessiter, si elle est importante, l'arrêt immédiat du réacteur.

Dans le second cas, il est également souhaitable de detecter les bulles d'argon dans le sodium de refroidissement du réacteur puisque ces bulles d'argon sont susceptibles de réduire de façon importante la capacité de refroidissement du sodium et donc de conduire à la formation de points chauds sur les assemblages combustibles qui sont préjudiciables à leur bonne tenue en service.

Dans le troisième cas ces bulles de vapeur formées par la cavitation d'une pompe vont, en implosant, entraîner des érosions préjudiciables à la vie de l'appareil.

On connaît des méthodes de détection des bulles de gaz, par exemple, dans le cas de l'hydrogène, la diffusion de cet hydrogène à travers une paroi en contact d'un côté avec le métal liquide et de l'autre côté, avec un milieu à très faible pression. Une telle méthode est cependant relativement compliquée à mettre en oeuvre et présente une sensibilité qui dépend de la température du sodium et surtout un temps de réponse long.

On connaît également des procédés de détection ou de mesure de corps étrangers dans un milieu homogène utilisant les ultrasons. On a par exemple utilisé les ultrasons pour mesurer la quantité de vapeur sous forme de bulles dans de l'eau à haute température et à haute pression contenue dans une enveloppe. Ce procédé n'est cependant pas transposable au cas des métaux liquides, puisqu'il utilise des mesures de vitesse d'ultrasons et qu'il nécessite la présence d'émetteurs ou récepteurs d'ultrasons au voisinage immédiat ou au contact de l'enveloppe renfermant le liquide ; ce procédé utilise en effet des mesures de temps de parcours des ondes ultrasonores, dans l'eau sous pression éventuellement chargée de bulles de vapeur.

Dans le cas des métaux liquides, en particulier dans le cas du sodium liquide de refroidissement

3

d'un réacteur nucléaire à neutrons rapides, la température de ce métal liquide peut atteindre des températures très supérieures à 400° qui sont transmises à la paroi de l'enveloppe renfermant le métal. Les émetteurs-récepteurs d'ultrasons pouvant fonctionner correctement à ces températures sont très coûteux et de fiabilité aléatoire.

Le but de l'invention est donc de proposer un procédé de détection par ultrasons de bulles de gaz dans un métal liquide contenu dans une enveloppe qui soit simple et très sensible, qui ait un faible temps de réponse et qui puisse être mis en oeuvre pour des métaux liquides à très hautes températures.

Dans ce but, le procédé suivant l'invention est caractérisé par le fait :

- qu'on envoie un faisceau d'ultrasons dans le métal liquide depuis un point d'émission situé à l'extérieur et à une certaine distance de l'enveloppe de façon à provoquer la propagation de ces ultrasons sur une certaine longueur à travers le métal liquide,

- qu'on capte le faisceau d'ultrasons à l'extérieur à une certaine distance de l'enveloppe, après qu'il ait traversé le métal liquide,

- qu'on compare l'amplitude des ondes ultrasonores captées à l'amplitude des ondes ultrasonores émises pour déterminer l'atténuation du faisceau ultrasonore produite par la traversée du métal liquide,

- et qu'on en déduit la présence de bulles de gaz dans le liquide si l'atténuation du faisceau d'ultrasons dépasse un certain seuil soit de façon continue, soit par impulsions brèves.

L'invention est également relative à un dispositif de détection constitué par au moins un transducteur d'ultrasons couplé à l'enveloppe renfermant le

4

métal liquide par l'intermédiaire d'un guide d'ondes lié métallurgiquement à l'enveloppe.

Afin de bien faire comprendre l'invention, on va maintenant décrire, à titre d'exemples non limitatifs, en se référant aux figures jointes en annexe, plusieurs modes de réalisation d'un dispositif suivant l'invention permettant la détection de bulles de gaz dans un métal liquide circulant dans une conduite.

La figure 1 est une vue schématique avec coupe partielle suivant l'axe de la conduite, d'un dispositif de détection de bulles de gaz, suivant un premier mode de réalisation.

La figure 1a est un diagramme montrant les variations au cours du temps de l'atténuation du faisceau ultrasonore utilisé pour la détection de bulles, dans le cas d'une production continue de bulles dans le métal liquide.

La figure 1b est un diagramme montrant les variations au cours du temps de l'atténuation du faisceau ultrasonore, dans le cas de la présence de bulles isolées dans le métal liquide.

La figure 2 est une vue schématique avec coupe partielle suivant l'axe de la conduite, d'un dispositif de détection suivant l'invention et suivant un second mode de réalisation.

La figure 3 est une vue schématique avec coupe partielle suivant l'axe de la conduite, d'un dispositif de détection suivant l'invention et suivant un troisième mode de réalisation.

La figure 3a est une vue suivant AA de la figure 3.

Les figures 4a, 4b, 4c et 4d sont des vues en coupe montrant la zone de liaison entre le guide d'ondes et la conduite dans laquelle passe le métal liquide.

La figure 5 est une vue en coupe transversale d'une conduite de métal liquide équipée d'un dispositif suivant l'invention et suivant une variante.

La figure 6 est une vue en coupe transversale partielle d'une conduite de métal liquide sur laquelle est fixé un guide d'ondes creux.

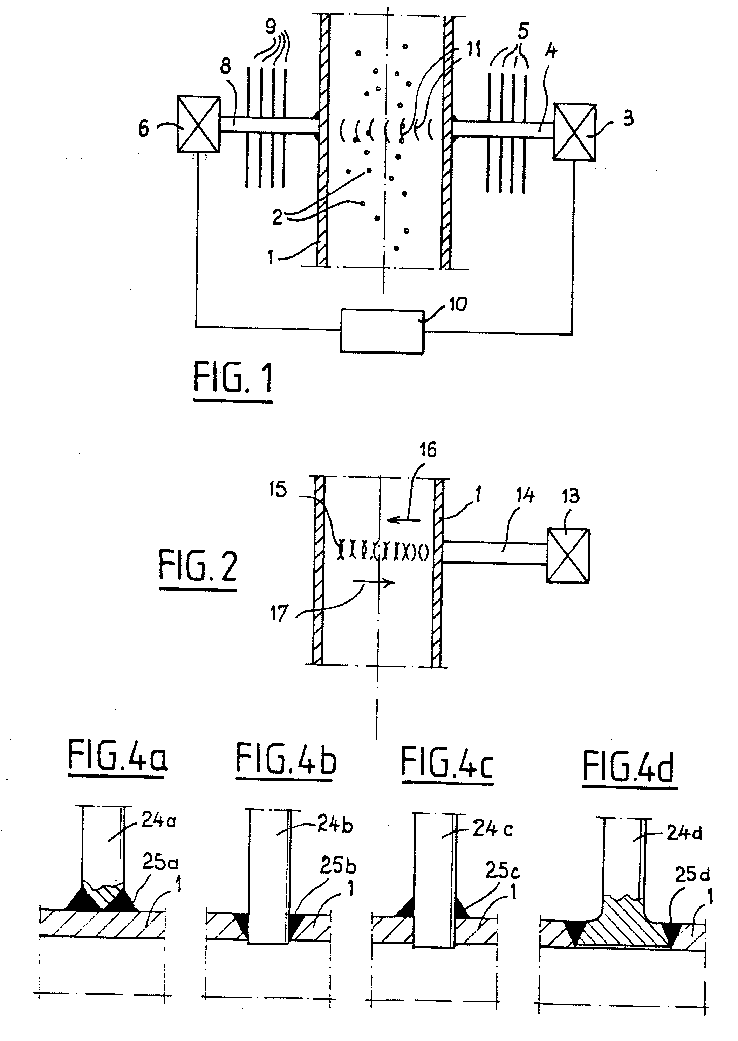

Sur la figure 1, on voit la conduite 1 dans laquelle circule le métal liquide qui est par exemple du sodium secondaire d'un réacteur nucléaire à neutrons rapides pouvant éventuellement contenir et entraîner des bulles d'hydrogène 2.

Le dispositif de détection de bulles de gaz comporte un émetteur d'ultrasons 3 couplé par l'intermédiaire d'un barreau métallique 4 à la paroi de la conduite 1 sur laquelle le barreau 4 est soudé à son extrémité opposée à son extrémité en contact avec l'émetteur d'ultrasons 3. Le barreau métallique 4 est entouré d'ailettes de refroidissement 5 permettant d'évacuer la chaleur transmise par la paroi de la conduite 1 et de limiter ainsi la température de l'extrémité du barreau 4 en contact avec l'émetteur 3.

Dans le cas où le métal liquide est du sodium primaire, sa température peut en effet atteindre 650°, en particulier dans le cas d'un fonctionnement en régime accidentel du réacteur. L'éloignement de l'émetteur 3 par rapport à la paroi de la conduite 1 et la présence d'ailettes permettent de limiter la température de la surface de contact entre l'émetteur et le guide d'ondes à un niveau autorisant l'utilisation d'un capteur ordinaire. Le dispositif de détection comporte également un récepteur 6 d'ondes ultrasonores couplé à la paroi de la conduite 1 par l'intermédiaire d'un guide d'ondes métallique 8 soudé sur la conduite; ce dernier peut être dans le prolongement du guide d'ondes 4. Le guide d'ondes 8 peut comporter aussi des ailettes de refroidissement 9 pour limiter sa température à son extrémité reliée au récepteur d'ultrasons 6. Une unité de traitement 10 reçoit les signaux électriques correspondant aux ondes ultrasonores transmises par le transducteur 3 et aux ondes ultrasonores reçues par le transducteur 6. L'unité de traitement 10 permet de comparer les amplitudes des signaux correspondant aux ondes ultrasonores émises et reçues respectivement, pour en déduire l'atténuation subie par les ondes 11 lors de leur propagation dans le métal liquide éventuellement chargé de bulles de gaz 2.

L'atténuation des ondes ultrasonores dans le métal liquide est considérablement accrue lorsque des bulles de gaz sont présentes dans ce métal. Comme il est visible sur les figures 1a et 1b, la présence d'un flux continu de bulles se traduit par une augmentation de l'atténuation qui se stabilise à une valeur pratiquement constante alors qu'une faible presence de bulles sous forme isolée se traduit par une succession d'impulsions brèves, sur la courbe d'attenuation. On determine donc le seuil d'attenuation a partir duquel on peut assurer que la présence de bulles dans le métal liquide est effective. Un module de comparaison intégré a l'unité de traitement 10 permet de déterminer si le seuil d'attenuation est atteint. Lorsque le seuil d'attenuation est atteint ou dépassé, un dispositif d'alarme ou d'affichage associe a l'unité de traitement 10 permet d'alerter l'opérateur responsable de l'installation.

Pour obtenir une detection optimale des bulles de gaz dans du sodium liquide, on utilisera une

7

fréquence généralement comprise entre 100 kHz et 10 MHz. En fait, l'atténuation dans le métal liquide chargé de bulles de gaz dépend, dans une certaine mesure, de la fréquence des ondes ultrasonores utilisées et de la taille des bulles présentes. On choisira donc une fréquence permettant d'obtenir la meilleure sensibilité possible, en fonction des caractéristiques de l'installation, de la nature du gaz susceptible de se former et d'être entraîné par le métal liquide et de la taille des bulles susceptibles de se former.

De toutes façons, dans le cas des circuits de sodium d'un réacteur nucléaire à neutrons rapides, la fréquence doit être choisie nettement supérieure aux fréquences propres de ce matériel. Pour satisfaire à cette exigence, on utilisera toujours des ondes d'une fréquence supérieure à 20kHz. Le procédé et le dispositif de détection selon l'invention sont extrèmement sensibles, en particulier lorsque la fréquence des ondes ultrasonores est choisie convenablement. C'est ainsi que dans du sodium liquide circulant dans une conduite d'un circuit de réacteur nucléaire à neutrons rapides, on a pu mesurer des atténuations de l'ordre de 6 décibels pour des taux de gaz de l'ordre de 5 % dans le liquide, avec des ondes ultrasonores à une fréquence de 500 kHz ; dans les mêmes conditions, on a pu mesurer des atténuations de l'ordre de 20 décibels pour des taux de gaz de 7 %, avec des ondes ultrasonores à la fréquence 2 MHz.

Dans le cas de générateurs de vapeur de grande taille du type de ceux qui équipent la centrale à neutrons rapides Super Phénix, compte tenu des débits de sodium, on peut, en utilisant le procédé et le dispositif suivant l'invention, détecter des fuites inférieures à 10 g/s d'eau par des mesures d'atténua-

8

tion d'ondes ultrasonores par les bulles d'hydrogène formées et entraînées dans la conduite de sortie de sodium du générateur de vapeur.

Sur la figure 2, on voit une variante de réalisation du dispositif de détection, le transducteur 13 étant constitué par un émetteur-récepteur d'ondes ultrasonores qui assure à la fois l'émission et la réception de ces ondes. Comme précédemment, ce transducteur 13 est relié à une unité de traitement non représentée qui permet de mesurer l'atténuation des ondes 15 dues à la propagation de ces ondes dans le métal liquide éventuellement chargé de bulles de gaz. Les ondes 15 se propagent tout d'abord dans le sens de la flèche 16, suivant une direction sensiblement diamètrale de la conduite 1, sont réfléchies par la paroi de la conduite 1 opposée au guide d'ondes 14 et sont renvoyées dans ce guide d'ondes 14 suivant le sens indiqué par la flèche 17 et suivant la même direction diamètrale qu'à l'aller. La disposition représentée à la figure 2 a l'avantage d'augmenter la longueur du parcours des ultrasons dans le métal liquide et donc l'atténuation et la sensibilité de la détection. Il est bien évident que le guide d'ondes 14 peut être équipé d'ailettes de refroidissement comme précédemment.

Sur les figures 3 et 3a, on voit un mode de réalisation du dispositif selon l'invention particulièrement adapté au cas d'une conduite dont le diamètre est relativement important (par exemple supérieur à 0,20 - 0,30 m), conduite comportant des changements de direction brusques tels que des coudes, ce qui est presque toujours le cas dans les circuits des réacteurs nucléaires à neutrons rapides. Dans le cas d'une conduite de grand diamètre comportant des coudes ou

9

sur laquelle sont montés divers appareils, la concentration des bulles peut en effet être essentiellement variable dans la section de la conduite. Il est alors souhaitable d'effectuer la mesure d'atténuation suivant plusieurs directions de la section, par exemple suivant plusieurs diamètres et/ou à plusieurs endroits suivant la longueur de la conduite. Le dispositif représenté sur les figures 3 et 3a comporte un premier ensemble de mesure 20 et un second ensemble de mesure 21 identiques à l'ensemble de mesure unique représenté sur la figure 1. Les guides d'ondes de ces deux ensembles de mesure sont fixés par soudure sur l'enveloppe de la conduite 1 de telle façon que les voies de mesure correspondantes 22 et 23 des ensembles 20 et 21 respectivement soient situées dans deux sections de la conduite 1 séparées par une certaines longueur. Ces voies de mesure sont d'autre part dirigées à 90° l'une par rapport à l'autre. Il est bien évident qu'on peut utiliser une unité de traitement commune pour les deux ensembles ou voies de mesure. Dans le cas d'une concentration de bulles dans une partie de la conduite, la détection sera évidemment meilleure en effectuant le balayage suivant deux diamètres et a des emplacements différents suivant la longueur de la conduite. On pourrait bien sûr multiplier le nombre d'ensembles ou voies de mesure disposés angulairement et espacés suivant la longueur de la conduite, pour augmenter la sensibilité et la fiabilité de la détection.

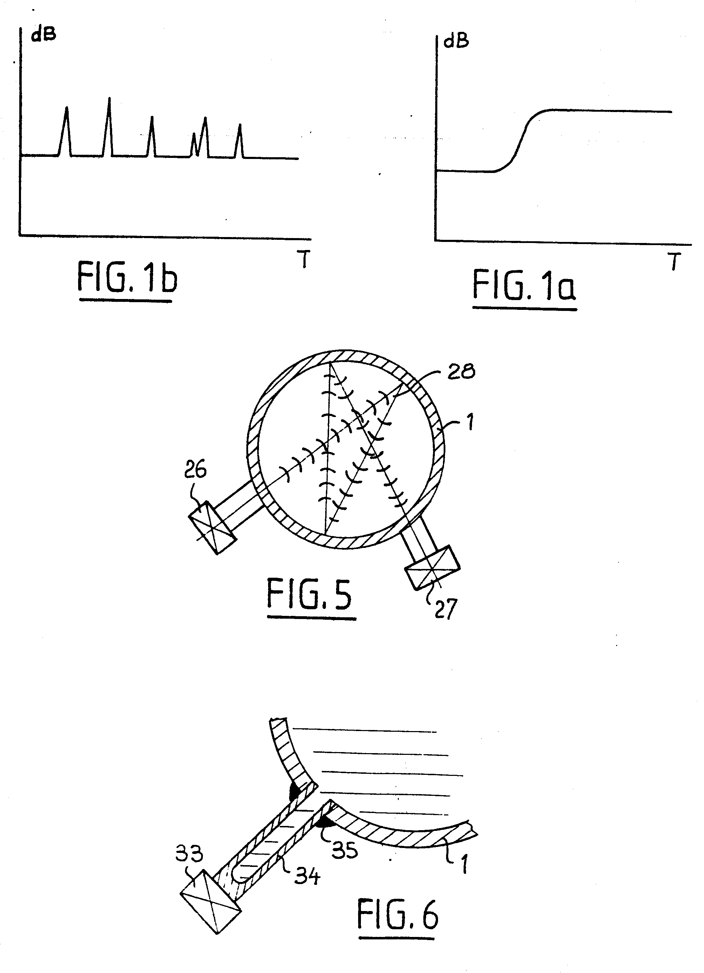

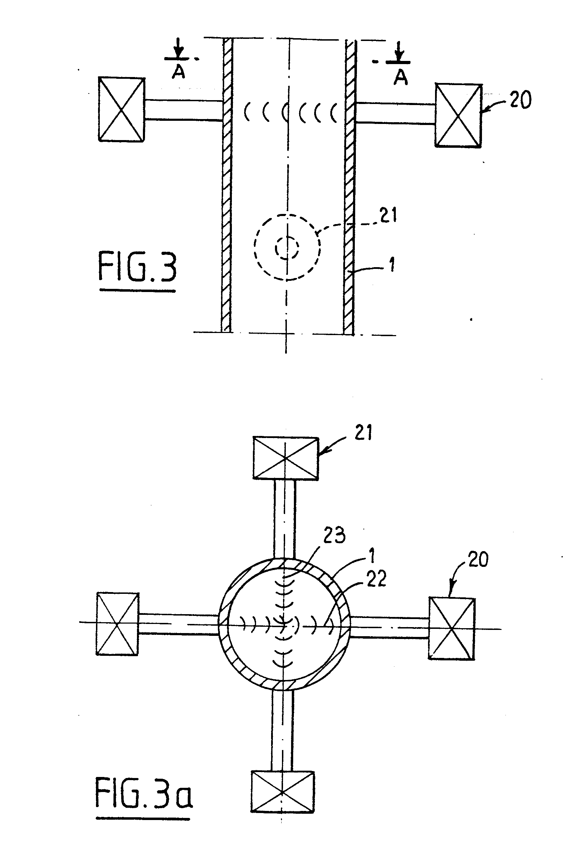

Il est aussi possible, comme il est visible sur la figure 5, de disposer les deux capteurs 26, 27 dans une configuration non alignée. Le train d'ondes 28 émis par le premier capteur 26 se réfléchit plusieurs fois sur la paroi interne de la conduite 1 et balaie ainsi une grande partie de la section avant d'être capté par le second capteur 27. Dans ce cas, les guides d'onde ne doivent pas être dirigés suivant un diamètre de la conduite 1.

D'autre part, puisque la fréquence permettant d'obtenir la meilleure sensibilité de détection dépend de la taille des bulles, il peut être intéressant, dans le cas du dispositif représenté aux figures 3 et 3a, d'alimenter chacun des ensembles successifs tels que 20, 21 de façon à obtenir une fréquence différente pour chacune des voies de mesure successives 22, 23.

Dans le cas où l'on utilise un seul ensemble de mesure, il peut être intéressant, pour la même raison, d'alimenter cet ensemble de façon à obtenir une fréquence des ondes ultrasonores variable dans le temps. Cette variation peut être continue ou incrémentale avec retours périodiques à une valeur déterminée.

Dans le cas où la source de gaz introduit dans le métal liquide est connue, par exemple dans le cas d'un générateur de vapeur situé sur le circuit secondaire d'un réacteur nucléaire a neutrons rapides, on effectuera l'étalonnage des ensembles de détection en disposant un premier ensemble en amont du générateur de vapeur, c'est-à-dire sur la conduite d'entrée de sodium dans le générateur de vapeur et un second ensemble en aval du générateur de vapeur, c'est-à-dire sur la conduite de sortie du sodium. La comparaison des attenuations mesurées a l'entree et a la sortie du générateur de vapeur permettra de déterminer de façon sûre la presence d'une fuite, dans le cas d'une différence sensiblement supérieure à un certain seuil prédéterminé, entre les atténuations mesurées.

Dans le cas d'une source de gaz non identifiée ou aléatoire, il peut être intéressant de placer en amont du dispositif de détection une source de gaz permettant de commander l'émission de bulles dans le métal liquide. Cette émission contrôlée et mesurée permet d'étalonner le dispositif de détection.

Sur les figures 4a, 4b, 4c et 4d, on a représenté quatre modes de jonction différents par soudure d'un guide d'un guide d'ondes 24a, 24b, 24c ou 24d respectivement, sur une conduite 1 destinée à recevoir du métal liquide.

Le guide d'ondes 24a est solidarisé à la surface externe de la conduite 1 par une soudure 25a.

Les guides d'ondes 24b, 24c et 24d sont traversants et liés à la paroi de la conduite 1, soit par une soudure pénétrante 25b occupant toute l'épaisseur de la paroi, soit par une soudure externe 24c, soit encore par une soudure bout à bout 25d entre une partie élargie du guide d'ondes 24d et la conduite 1.

Les modes de jonction représentés sur les figures 4b et 4d sont préférables dans le cas de conduites subissant des sollicitations importantes.

Les conduites pour métaux liquides, en particulier pour le sodium utilisé dans les réacteurs nucléaires à neutrons rapides, sont souvent réalisées en acier inoxydable austénitique. Lorsque le guide d'ondes a une partie qui vient directement en contact avec le métal liquide, cette partie doit également être en acier inoxydable pour éviter sa corrosion et pour permettre le soudage avec la conduite en acier inoxydable austénitique. Cependant, la propagation des ondes ultrasonores n'est pas aussi bonne dans l'acier inoxydable austénitique que dans l'acier au carbone. On peut donc réaliser le guide d'ondes ou tout au moins la partie de ce guide d'ondes venant en contact avec le

12

métal liquide, sous forme creuse, en particulier tubulaire, comme il est visible sur la figure 6 ; le liquide vient alors remplir la cavité du guide d'ondes 34 en contact avec le capteur 33. On peut également réaliser la partie courante du guide d'ondes en acier au carbone et prévoir, à l'extrémité de ce guide d'ondes dont on effectuera la jonction avec la conduite, une pastille en acier inoxydable rendue solidaire de la partie courante du guide d'ondes par une soudure hétérogène ou par une brasure.

Les guides d'ondes utilisables pour la détection de bulles de gaz suivant le procédé de l'invention ne permettent généralement pas d'obtenir un faisceau dont le diamètre est important, c'est la raison pour laquelle il peut être nécessaire d'utiliser plusieurs ensembles de mesure pour effectuer un balayage plus complet de la section ou de la longueur de la conduite. Cependant, il est possible de modifier la forme du faisceau d'ondes ultrasonores en prévoyant un guide d'ondes venant en contact avec le métal liquide par une surface de forme variee. Dans le cas d'une surface plane, on obtient ainsi un faisceau parallele et dans le cas d'une surface arrondie et concave, un faisceau convergent. On peut ainsi améliorer la sensibilité de détection suivant la localisation et le cheminement des bulles dans la conduite.

Il apparaît donc que le procédé et le dispositif suivant l'invention permettent une détection sensible et fiable de bulles de gaz dans un métal liquide quelconque, éventuellement a tres haute température.

Le procédé et le dispositif suivant l'invention ne sont pas limités aux modes de réalisation qui ont été décrits.

13

On peut concevoir d'autres formes de réalisation des guides d'ondes et de leur jonction avec l'enveloppe contenant le métal liquide et d'autres dispositions d'ensembles de mesure suivant la circonférence ou suivant la longueur de l'enveloppe renfermant le métal liquide.

On peut également concevoir d'autres procédés et dispositifs de traitement des signaux des transducteurs ultrasonores pour la détermination de l'atténuation des ultrasons.

Le procédé et le dispositif suivant l'invention peuvent être utilisés pour la détection de bulles de gaz dans des enveloppes de formes différentes de celle d'une conduite circulaire où le métal liquide est en circulation. Ce procédé et ce dispositif s'appliquent dans le cas de réservoirs ou enceintes renfermant du métal liquide stagnant ou immobile.

Enfin, le procédé et le dispositif suivant l'invention sont utilisables sur d'autres installations que les réacteurs nucléaires à neutrons rapides et dans d'autres domaines que l'industrie nucléaire. De façon générale, ce procédé et ce dispositif s'appliquent dans toute forme d'industries utilisant des métaux liquides suceptibles de renfermer des bulles de gaz, que ce gaz soit de nature quelconque ou la propre vapeur du métal liquide.

14

REVENDICATIONS

1.- Procédé de détection par ultrasons de bulles de gaz (2) dans un métal liquide contenu dans une enveloppe (1) caractérisé par le fait :

- qu'on envoie un faisceau d'ultrasons dans le métal liquide depuis un point d'émission (3) situé à l'extérieur et à une certaine distance de l'enveloppe (1) de façon à provoquer la propagation de ces ultrasons sur une certaine longueur à travers le métal liquide,

- qu'on capte le faisceau d'ultrasons à l'extérieur et à une certaine distance de l'enveloppe (1), après qu'il ait traversé le métal liquide,

- qu'on compare l'amplitude des ondes ultrasonores captées à l'amplitude des ondes ultrasonores émises pour déterminer l'atténuation du faisceau d'ondes ultrasonores produite par la traversée du métal liquide,

- et qu'on en déduit la présence de bulles de gaz (2) dans le liquide si l'atténuation du faisceau d'ondes ultrasonores dépasse un certain seuil, soit de façon continue, soit par impulsions brèves.

2.- Procédé de détection suivant la revendication 1, caractérisé par le fait que la fréquence des ondes ultrasonores est supérieure à 20 kHz.

3.- Procédé de détection suivant la revendication 2, caractérisé par le fait que la fréquence des ondes ultrasonores est comprise entre 100 kHz et 10 MHz.

4.- Procédé de détection suivant l'une quelconque des revendications 1, 2 et 3, caractérisé par le fait que la fréquence des ondes ultrasonores et variable dans le temps.

5.- Procédé de détection suivant la revendication 4, caractérisé par le fait que la fréquence des ondes ultrasonores varie de façon incrémentale, avec un passage périodique à au moins une fréquence prédéterminée.

6.- Dispositif de détection par ultrasons de bulles de gaz (2) dans un métal liquide contenu dans une enveloppe (1), caractérisé par le fait qu'il comporte au moins un transducteur d'ultrasons (3, 6, 13) couplé à l'enveloppe (1) par un guide d'ondes (4, 8, 14) d'une certaine longueur lié métallurgiquement à l'enveloppe (1) et un dispositif (10) de mesure d'atténuation des ultrasons associé aux transducteurs (3, 6, 13).

7.- Dispositif de détection suivant la revendication 6, caractérisé par le fait qu'il comporte un émetteur d'ultrasons (3) couplé à l'enveloppe (1) par un guide d'ondes (4) et un récepteur d'ultrasons (6) couplé à l'enveloppe (1) par un guide d'ondes (8), avec interposition de métal liquide entre les extrémités des guides d'ondes (4, 8), liés à l'enveloppe (1).

8.- Dispositif de détection suivant la revendication 6, caractérisé par le fait qu'il comporte un émetteur-récepteur d'ultrasons (13) couplé à l'enveloppe (1) par un guide d'ondes (14).

9.- Dispositif de détection suivant la revendication 6, caractérisé par le fait qu'il comporte au moins deux ensembles de mesure (20, 21) comportant chacun au moins un transducteur d'ultrasons couplé à l'enveloppe (1) par un guide d'ondes.

10.- Dispositif de détection suivant la revendication 9, dans le cas où l'enveloppe (1) est une conduite tubulaire dans laquelle circule le métal liquide, caractérisé par le fait que les ensembles de mesure (20, 21) comportent des voies de mesure (22, 23) suivant lesquelles se propagent les ultrasons, disposées suivant au moins deux directions traversant la conduite (1) faisant un certain angle et espacées suivant la longueur de la conduite (1).

11.- Dispositif de détection suivant la revendication 10, caractérisé par le fait que les voies de mesure (22, 23) sont disposées à 90° et suivant deux diamètres de la conduite (1).

12.- Dispositif de détection suivant l'une quelconque des revendications 10 et 11, caractérisé par le fait que les ensembles de mesure successifs (20, 21) sont alimentés en ondes ultrasonores à des fréquences différentes.

13.- Dispositif de détection suivant la revendication 7, caractérisé par le fait qu'il comporte un émetteur d'ultrasons (26) couplé à l'enveloppe (1) par un guide d'ondes et un récepteur d'ultrasons (27) couplé à l'enveloppe (1) par un guide d'ondes non aligné avec le guide d'onde du récepteur (27), les guides d'ondes étant placés de façon que l'onde ultrasonique (28) soit reçue par le récepteur (27) après au moins une réflexion sur la surface interne de l'enveloppe (1).

14.- Dispositif de détection suivant l'une quelconque des revendications 6 a 13, caractérisé par le fait que le guide d'ondes (4, 14, 24b, 24c, 24d) est traversant par rapport à la paroi de l'enveloppe (1).

15.- Dispositif de détection suivant la revendication 14, caractérisé par le fait que l'extrémité traversante du guide d'ondes (24b, 24c, 24d) en contact avec le métal liquide est plane.

16.- Dispositif de détection suivant la revendication 14, caractérisé par le fait que l'extrémité traversante du guide d'ondes (24b, 24c, 24d) en contact avec le métal liquide est arrondi.

17

17.- Dispositif de détection suivant la revendication 14, caractérisé par le fait que l'extrémité traversante du guide d'ondes (34) est creuse de façon à se remplir de métal liquide.

FIG. 1

FIG. 2

FIG.4a   FIG.4b   FIG.4c   FIG.4d

dB

FIG. 1b

T

dB

FIG. 1a

T

FIG. 5

28

1

26

27

FIG. 6

33

34

35

1

FIG.3

FIG.3a

0221796

Office européen
des brevets

**RAPPORT DE RECHERCHE EUROPEENNE**

Numéro de la demande **0221796**

EP 86 40 2116

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl.4) |
|---|---|---|---|
| Y | FR-A-2 288 979 (WESTINGHOUSE ELECTRIC) <br> * Page 1, ligne 30 - page 2, ligne 36 * | 1,6,7 | G 01 N 29/02 <br> G 01 N 33/20 |
| | --- | | |
| Y | PATENTS ABSTRACTS OF JAPAN, vol. 7, no. 54, (P-180)[1199], 4 mars 1983; & JP-A-57 200 856 (HITACHI SEISAKUSHO K.K.) 09.12.1982 <br> * Résumé * | 1,6,7 | |
| | --- | | |
| A | DE-A-1 473 707 (THE BRITISSH ALUMINIUM CO.) <br> * Page 2, ligne 10 - page 6, ligne 4 * | 1-3,6, 7 | |
| | --- | | |
| A | FR-A-2 404 850 (COMMISSARIAT A L'ENERGIE ATOMIQUE) <br> * Page 2, ligne 5 - page 3, ligne 39 * | 1,6,14 | **DOMAINES TECHNIQUES RECHERCHES (Int. Cl.4)** <br><br> G 01 N 29/00 <br> G 01 N 33/00 |
| | ----- | | |

Le présent rapport de recherche a été établi pour toutes les revendications

| Lieu de la recherche | Date d achevement de la recherche | Examinateur |
|---|---|---|
| LA HAYE | 19-01-1987 | BINDON C.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : theorie ou principe à la base de l invention
E : document de brevet anterieur, mais publie a la date de dépôt ou apres cette date
D : cité dans la demande
L : cité pour d'autres raisons

& : membre de la même famille, document correspondant

OEB Form 1503 03 82